# EUROPEAN PATENT APPLICATION

(11) **EP 4 242 197 A1**
(43) Date of publication of application: **13.09.2023**
(21) Application number: 22217221.5
(22) Date of filing: 29.12.2022
(51) Int. Cl.: C07C 69/96, C08F 283/00

(54) **CARBONATE-CONTAINING UNSATURATED COMPOUND, MANUFACTURING METHOD THEREOF, CROSSLINKED PRODUCT PREPARED THEREBY AND METHOD FOR DEGRADING CROSSLINKED PRODUCT**

(30) Priority: 07.03.2022 TW 111108215
(71) Applicant: Swancor Innovation & Incubation Co., Ltd., Nantou City, Nantou County 540028 (TW)
(72) Inventor: WANG, Meng-Wei, 54066 Nantou City (TW); CHEN, Chun-An, 54066 Nantou City (TW); CHANG, Meng-Ting, 54066 Nantou City (TW); CHANG, Chia-Hao, 54066 Nantou City (TW); CHENG, Ming-Yao, 54066 Nantou City (TW)
(74) Representative: D Young & Co LLP

(57) **Abstract**

A carbonate-containing unsaturated compound and a manufacturing method for a carbonate-containing unsaturated compound, a crosslinked product and a method for degrading a crosslinked product are provided. The carbonate-containing unsaturated compound includes a structure represented by formula (I) or formula (II). Formula (I) and formula (II) are defined as in the specification.

## Description

### BACKGROUND

### Technical Field

The present disclosure relates to an unsaturated compound, a manufacturing method thereof, a crosslinked product prepared thereby and a method for degrading a crosslinked product. More particularly, the present disclosure relates to a carbonate-containing unsaturated compound, a manufacturing method thereof, a crosslinked product prepared thereby and a method for degrading a crosslinked product.

### Description of Related Art

The thermosetting materials have the characteristics of good processability before curing, and have the excellent thermal stability, the mechanical strength and the chemical resistance after cross-linking and curing. Therefore, the thermosetting materials are widely used in the various fields, and often used in the fiber composite materials with the high strength and lightweight requirements. However, due to the characteristics of non-melt-processable and good chemical resistance of the thermosetting materials, also causing that it is difficult to recycle and reuse them after waste. Moreover, the combustion of fiber composite materials is easy to shorten the life of the incineration equipment and cause the problem of large amount of waste. Therefore, how to deal with the waste thermosetting materials is an improvement goal in today's environmental issues.

At present, the vinyl ester resin and the unsaturated polyester resin are commonly used thermosetting materials in the industry, which are widely used in the flied, such as coating, transportation and construction, etc. Due to the booming development of automobiles, ships, or other mass transportation industries, the application market thereof continues to expand. For the applications and developers, the disposal of resin waste has become a major issue that has to be faced, so that the industry is eager to know the technologies that can decompose or recycle the resin waste.

The current research trend uses acid aqueous solutions or alkaline aqueous solutions for degradation, which will lead to the problems such as subsequent treatment of the large amount of wastewater, and it is not friendly to the industrial applications and the environment. However, in terms of the current technology, the usage of the organic substance for degradation is limited by the limited content of the structural ester groups, and a relatively high degradation temperature must be applied, which has the disadvantages of energy consumption and poor efficiency, so there are still considerable application defects.

Therefore, how to synthesize the crosslinked product with the chemical degradability and can reuse the waste, which is the goal of the relevant industry.

### SUMMARY

According to one aspect of the present disclosure, a carbonate-containing unsaturated compound is provided. The carbonate-containing unsaturated compound includes a structure represented by formula (I) or formula (II): 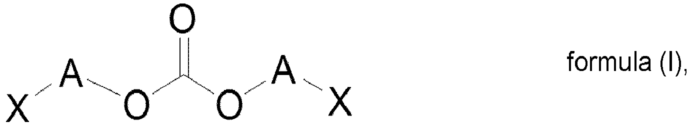 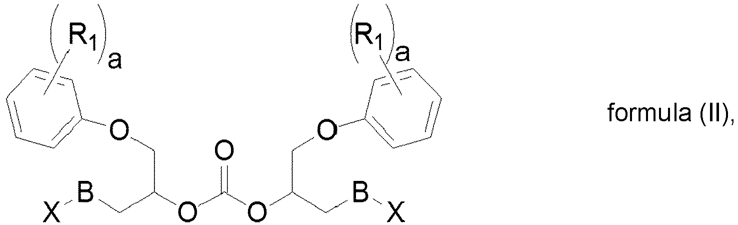
wherein X is a vinyl group, an allyl group, an acrylate or a methacrylate. R₁ is an alkyl group of 1 to 4 carbon atoms, a methoxy group, a nitro group or a halogen atom, and a is an integer from 0 to 5. A is an alkyl group of 1 to 12 carbon atoms, an alkoxy group of 1 to 12 carbon atoms or a structure represented by formula (i): 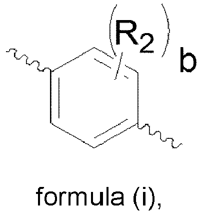
wherein R₂ is the alkyl group of 1 to 4 carbon atoms, the methoxy group, the nitro group or the halogen atom, and b is an integer from 0 to 4. B is the alkyl group of 1 to 12 carbon atoms, the alkoxy group of 1 to 12 carbon atoms, the structure represented by formula (i), or a structure represented by formula (ii) or formula (iii): 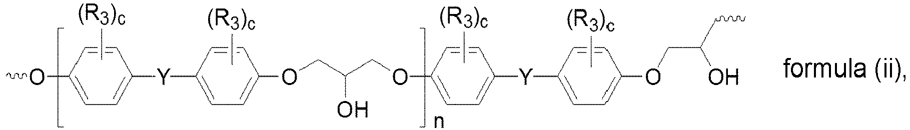 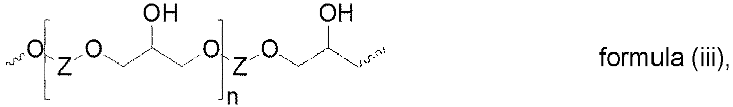
wherein R₃ is the alkyl group of 1 to 4 carbon atoms, the methoxy group, the nitro group or the halogen atom, and c is an integer from 0 to 4. Y is a single bond, the alkyl group of 1 to 12 carbon atoms, a cycloalkyl group of 3 to 12 carbon atoms, an oxygen atom, a sulfur atom, a sulfonyl group, a thionyl group, an acyl group, a fluorenyl group or a hexafluoropropane group. Z is the alkyl group of 1 to 12 carbon atoms, the alkoxy group of 1 to 12 carbon atoms, an otho-phenylene group, a meta-phenylene group or a para-phenylene group. n is an integer from 0 to 10.

According to another aspect of the present disclosure, a manufacturing method for a carbonate-containing unsaturated compound includes steps as follows. A carbonate-containing compound is provided, wherein the carbonate-containing compound is a dimethyl carbonate or a diphenyl carbonate. An unsaturated double bond-containing compound is provided, wherein the unsaturated double bond-containing compound is an unsaturated double bond-containing monofunctional alcohol compound or an unsaturated double bond-containing monofunctional epoxy compound. A catalyzing step is performed, wherein the carbonate-containing compound is reacted with the unsaturated double bond-containing compound by using a catalyst to obtain a carbonate-containing unsaturated compound, which includes a structure represented by formula (I) or formula (II): 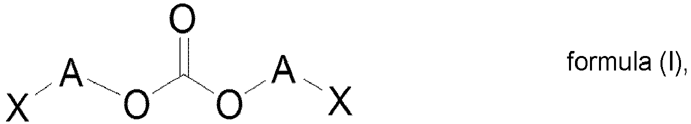 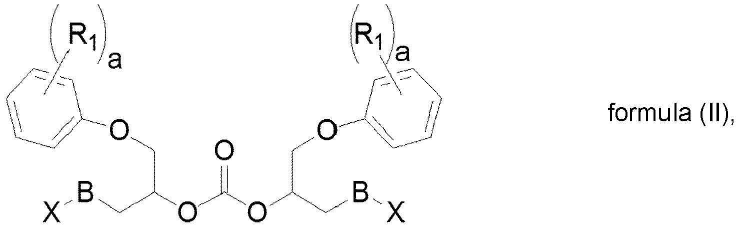
wherein X is a vinyl group, an allyl group, an acrylate or a methacrylate. R₁ is an alkyl group of 1 to 4 carbon atoms, a methoxy group, a nitro group or a halogen atom, and a is an integer from 0 to 5. A is an alkyl group of 1 to 12 carbon atoms, an alkoxy group of 1 to 12 carbon atoms or a structure represented by formula (i): 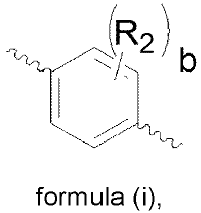
wherein R₂ is the alkyl group of 1 to 4 carbon atoms, the methoxy group, the nitro group or the halogen atom, and b is an integer from 0 to 4. B is the alkyl group of 1 to 12 carbon atoms, the alkoxy group of 1 to 12 carbon atoms, the structure represented by formula (i), or a structure represented by formula (ii) or formula (iii): 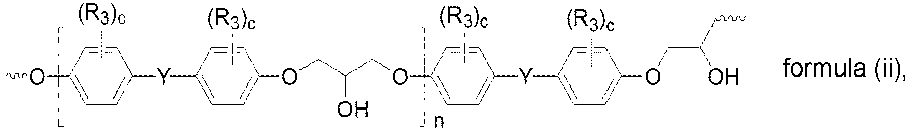 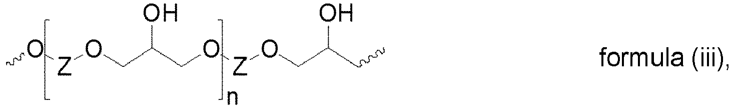
wherein R₃ is the alkyl group of 1 to 4 carbon atoms, the methoxy group, the nitro group or the halogen atom, and c is an integer from 0 to 4. Y is a single bond, the alkyl group of 1 to 12 carbon atoms, a cycloalkyl group of 3 to 12 carbon atoms, an oxygen atom, a sulfur atom, a sulfonyl group, a thionyl group, an acyl group, a fluorenyl group or a hexafluoropropane group. Z is the alkyl group of 1 to 12 carbon atoms, the alkoxy group of 1 to 12 carbon atoms, an otho-phenylene group, a meta-phenylene group or a para-phenylene group. n is an integer from 0 to 10.

According to the manufacturing method for the carbonate-containing unsaturated compound of the foregoing aspect, the catalyst can be an ionic liquid or an organic base.

According to the manufacturing method for the carbonate-containing unsaturated compound of the foregoing aspect, an equivalence ratio of the carbonate-containing compound to the unsaturated double bond-containing compound can be 0.8 to 1.2.

According to further another aspect of the present disclosure, a manufacturing method for a carbonate-containing unsaturated compound includes steps as follows. A catalyzing step is performed, wherein a diphenyl carbonate is reacted with a bifunctional epoxy compound by using a catalyst to obtain a reactant. An adding step is performed, wherein an acrylic acid or a methacrylic acid is added in the reactant to obtain a carbonate-containing unsaturated compound, which includes a structure represented by formula (II): 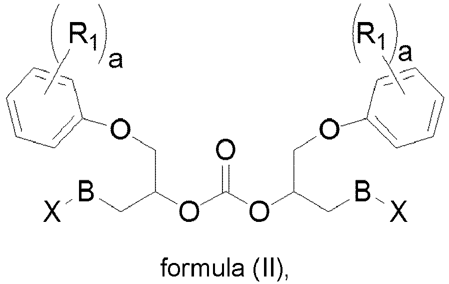
wherein X is a vinyl group, an allyl group, an acrylate or a methacrylate. R₁ is an alkyl group of 1 to 4 carbon atoms, a methoxy group, a nitro group or a halogen atom, and a is an integer from 0 to 5. B is an alkyl group of 1 to 12 carbon atoms, an alkoxy group of 1 to 12 carbon atoms, a structure represented by formula (i), formula (ii) or formula (iii): 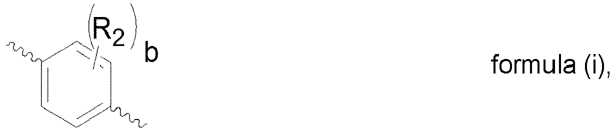 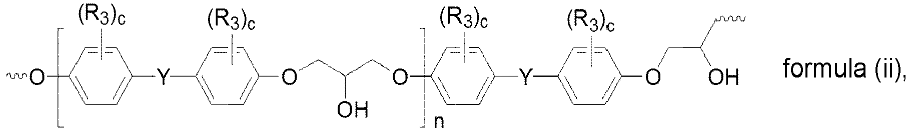 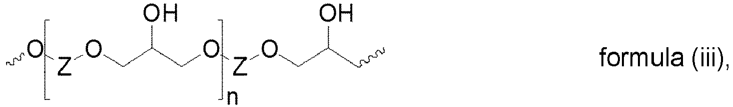
wherein R₂ is the alkyl group of 1 to 4 carbon atoms, the methoxy group, the nitro group or the halogen atom, and b is an integer from 0 to 4. R₃ is the alkyl group of 1 to 4 carbon atoms, the methoxy group, the nitro group or the halogen atom, and c is an integer from 0 to 4. Y is a single bond, the alkyl group of 1 to 12 carbon atoms, a cycloalkyl group of 3 to 12 carbon atoms, an oxygen atom, a sulfur atom, a sulfonyl group, a thionyl group, an acyl group, a fluorenyl group or a hexafluoropropane group. Z is the alkyl group of 1 to 12 carbon atoms, the alkoxy group of 1 to 12 carbon atoms, an otho-phenylene group, a meta-phenylene group or a para-phenylene group. n is an integer from 0 to 10.

According to the manufacturing method for the carbonate-containing unsaturated compound of the foregoing aspect, the catalyst is an organic base, and an equivalence ratio of the bifunctional epoxy compound to the diphenyl carbonate can be 2.0 to 8.0.

According to still another aspect of the present disclosure, a crosslinked product is provided. The crosslinked product is obtained by performing a curing reaction with the carbonate-containing unsaturated compound according to the aforementioned aspect.

According to the crosslinked product of the foregoing aspect, the curing reaction can be completed by heating after the carbonate-containing unsaturated compound added to a resin.

According to the crosslinked product of the foregoing aspect, the resin can be an unsaturated polyester resin or a vinyl ester resin.

According to the crosslinked product of the foregoing aspect, an added amount of the carbonate-containing unsaturated compound can be ranged from 3% by weight to 20% by weight of an amount of the resin.

According to yet another aspect of the present disclosure, a method for degrading a crosslinked product includes steps as follows. The crosslinked product according to the aforementioned aspect is provided. A degrading step is performed, wherein the crosslinked product is degraded by using an amine compound as a degradation agent.

### BRIEF DESCRIPTION OF THE DRAWINGS

The present disclosure can be more fully understood by reading the following detailed description of the embodiment, with reference made to the accompanying drawings as follows:
Fig. 1 is a flow chart of a manufacturing method for a carbonate-containing unsaturated compound according to one embodiment of the present disclosure.
Fig. 2 is a flow chart of a manufacturing method for a carbonate-containing unsaturated compound according to another embodiment of the present disclosure.
Fig. 3 is a flow chart of a manufacturing method for a crosslinked product according to further another embodiment of the present disclosure.
Fig. 4 is a flow chart of a method for degrading a crosslinked product according to yet another embodiment of the present disclosure.

### DETAILED DESCRIPTION

The present disclosure will be further exemplified by the following specific embodiments. However, the embodiments can be applied to various inventive concepts and can be embodied in various specific ranges. The specific embodiments are only for the purposes of description, and are not limited to these practical details thereof.

In the present disclosure, the compound structure can be represented by a skeleton formula, and the representation can omit the carbon atom, the hydrogen atom and the carbon-hydrogen bond. In the case that the functional group is depicted clearly in the structural formula, the depicted one is preferred.

In the present disclosure, in order to concise and smooth, "carbonate-containing unsaturated compound, comprising a structure represented by formula (I)" can be represented as a carbonate-containing unsaturated compound represented by formula (I) or a carbonate-containing unsaturated compound (I) in some cases, and the other compounds or groups can be represented in the same manner.

### <A Carbonate-Containing Unsaturated Compound>

A carbonate-containing unsaturated compound is provided of the present disclosure, which includes a structure represented by formula (I) or formula (II): 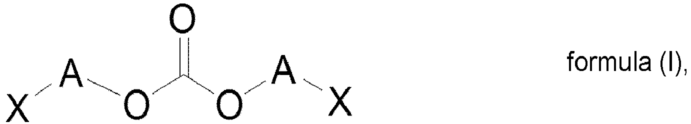 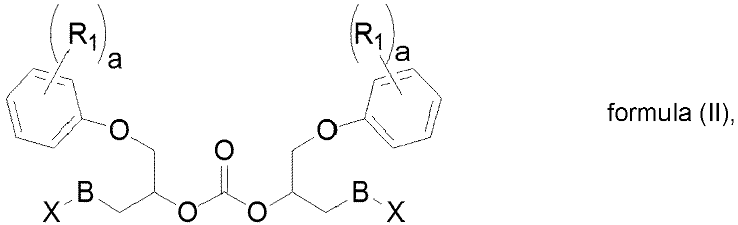
wherein X is a vinyl group, an allay group, an acrylate or a methacrylate. R₁ is an alkyl group of 1 to 4 carbon atoms, a methoxy group, a nitro group or a halogen atom, and a is an integer from 0 to 5. A is an alkyl group of 1 to 12 carbon atoms, an alkoxy group of 1 to 12 carbon atoms or a structure represented by formula (i): 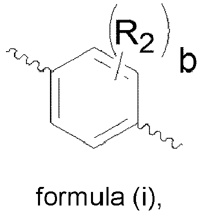
wherein R₂ is the alkyl group of 1 to 4 carbon atoms, the methoxy group, the nitro group or the halogen atom, and b is an integer from 0 to 4. B is the alkyl group of 1 to 12 carbon atoms, the alkoxy group of 1 to 12 carbon atoms, the structure represented by formula (i), or a structure represented by formula (ii) or formula (iii): 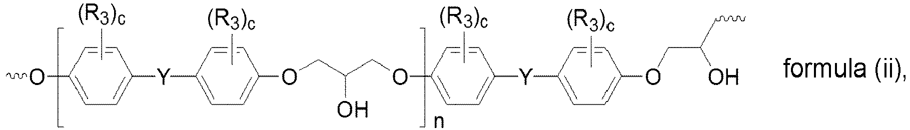 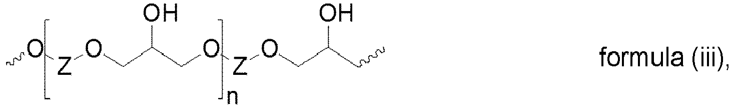
wherein R₃ is the alkyl group of 1 to 4 carbon atoms, the methoxy group, the nitro group or the halogen atom, and c is an integer from 0 to 4. Y is a single bond, the alkyl group of 1 to 12 carbon atoms, a cycloalkyl group of 3 to 12 carbon atoms, an oxygen atom, a sulfur atom, a sulfonyl group, a thionyl group, an acyl group, a fluorenyl group or a hexafluoropropane group. Z is the alkyl group of 1 to 12 carbon atoms, the alkoxy group of 1 to 12 carbon atoms, an otho-phenylene group, a meta-phenylene group or a para-phenylene group, and n is an integer from 0 to 10.

Therefore, the carbonate-containing unsaturated compound of the present disclosure is given the thermosetting product degradability by introducing the carbonate ester group structure, and the purpose of recycling and degrading the material can be achieved in the future.

### <A Manufacturing Method for a Carbonate-Containing Unsaturated Compound>

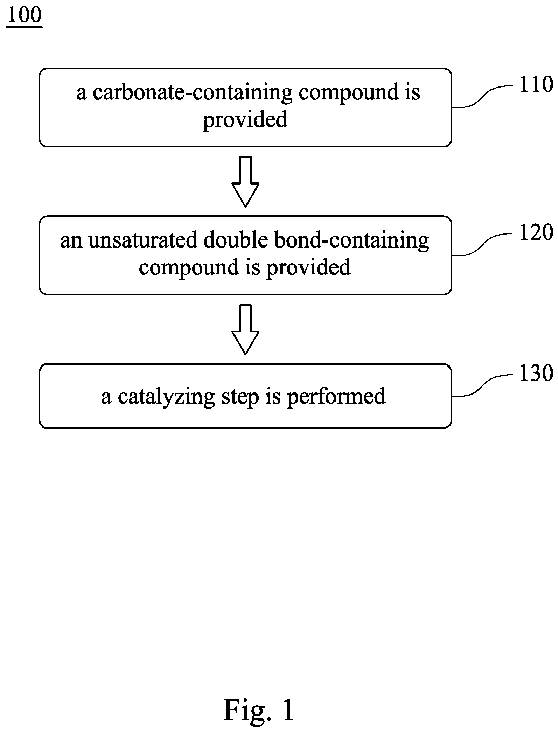

Reference is made to Fig. 1, which is a flow chart of a manufacturing method for a carbonate-containing unsaturated compound 100 according to one embodiment of the present disclosure. In Fig. 1, the manufacturing method for the carbonate-containing unsaturated compound 100 includes a step 110, a step 120 and a step 130.

In the step 110, a carbonate-containing compound is provided, wherein the carbonate-containing compound is a dimethyl carbonate (DMC) or a diphenyl carbonate (DPC).

In the step 120, an unsaturated double bond-containing compound is provided, wherein the unsaturated double bond-containing compound is an unsaturated double bond-containing monofunctional alcohol compound or an unsaturated double bond-containing monofunctional epoxy compound.

In the step 130, a catalyzing step is performed, wherein the carbonate-containing compound is reacted with the unsaturated double bond-containing compound by using a catalyst to obtain a carbonate-containing unsaturated compound, which includes a structure represented by formula (I) or formula (II): 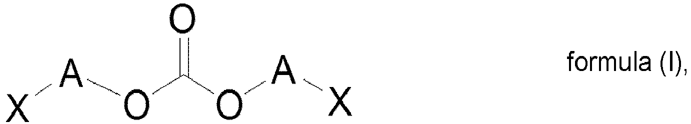 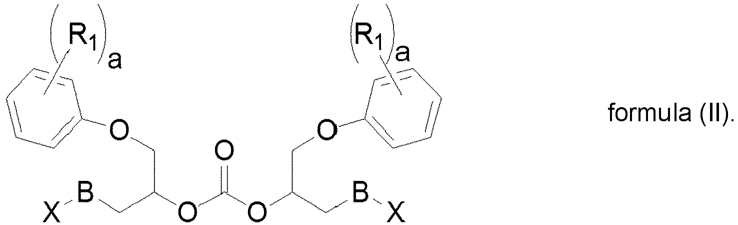 The definition of X, A, B, R₁ and a can refer to the aforementioned paragraph, and will not be described herein. The aforementioned catalyst can be an ionic liquid or an organic base.

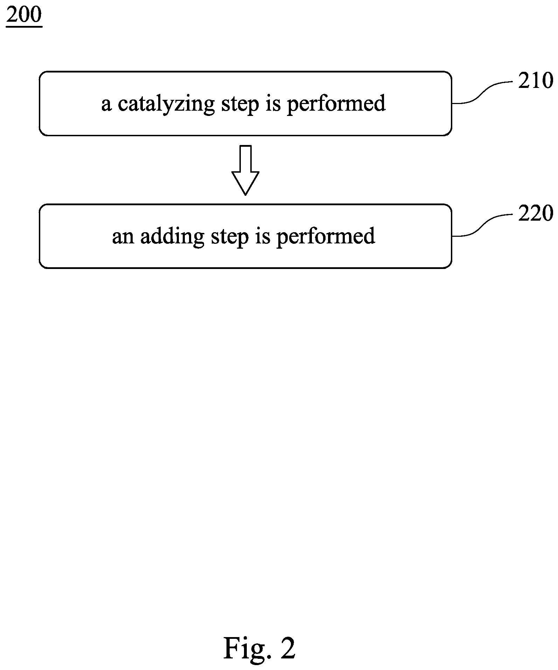

Reference is made to Fig. 2, which is a flow chart of a manufacturing method for a carbonate-containing unsaturated compound 200 according to another embodiment of the present disclosure. In Fig. 2, the manufacturing method for the carbonate-containing unsaturated compound 200 includes a step 210 and a step 220.

In the step 210, a catalyzing step is performed, wherein a diphenyl carbonate is reacted with a bifunctional epoxy compound by using a catalyst to obtain a reactant. The aforementioned catalyst can be an organic base.

In the step 220, an adding step is performed, wherein an acrylic acid or a methacrylic acid is added in the reactant to obtain a carbonate-containing unsaturated compound, which includes a structure represented by formula (II): 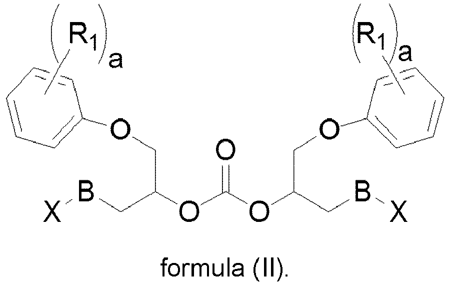 The definition of X, B, R₁ and a can refer to the aforementioned paragraph, and will not be described herein.

Specifically, when the carbonate-containing unsaturated compound includes the structure represented by formula (I), the preparation method thereof is to react the dimethyl carbonate with the unsaturated double bond-containing monofunctional alcohol compound at an equivalent ratio of 0.8 to 1.2, and the ionic liquid is used as the catalyst. An added amount of the catalyst can be ranged from 0.01 % by weight to 2% by weight of a total content of the reactant, and a reaction temperature is 60°C to 90°C.

Furthermore, when the carbonate-containing unsaturated compound includes the structure represented by formula (II), which has the two preparation methods. The first preparation method is to react the diphenyl carbonate with the unsaturated double bond-containing monofunctional epoxy compound at an equivalent ratio of 0.8 to 1.2, and the organic base is used as the catalyst. An added amount of the catalyst can be ranged from 0.01% by weight to 2% by weight of a content of the unsaturated double bond-containing monofunctional epoxy compound, and a reaction temperature is 80°C to 140°C. The second preparation method is to react the bifunctional epoxy compound with the diphenyl carbonate at an equivalent ratio of 2.0 to 8.0, and the organic base is used as the catalyst. An added amount of the catalyst can be ranged from 0.01% by weight to 2% by weight of a content of the bifunctional epoxy compound, and then the acrylic acid or the methacrylic acid is added for reaction, wherein an equivalence ratio of the acrylic acid or the methacrylic acid to the bifunctional epoxy compound is 0.4 to 0.6, and a reaction temperature is 80°C to 140°C.

### <A Crosslinked Product>

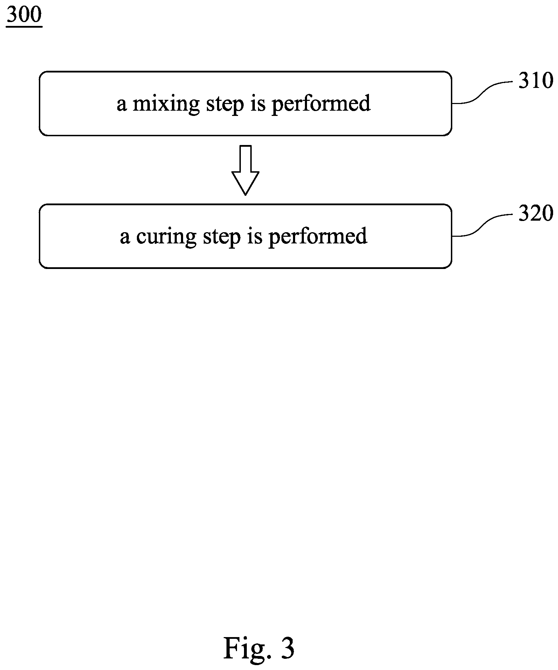

A crosslinked product is further provided of the present disclosure, which is obtained by performing a curing reaction with the carbonate-containing unsaturated compound. The aforementioned curing reaction is referred with Fig. 3, which is a flow chart of a manufacturing method for a crosslinked product 300 according to further another embodiment of the present disclosure. In Fig. 3, the manufacturing method for the crosslinked product 300 includes a step 310 and a step 320.

In the step 310, a mixing step is performed, wherein the carbonate-containing unsaturated compound is added to a resin. Specifically, an added amount of the carbonate-containing unsaturated compound is ranged from 3% by weight to 20% by weight of an amount of the resin, which can improve the degradability of the crosslinked product without affecting the basic physical properties. The detail of the carbonate-containing unsaturated compound can refer to the aforementioned paragraph, and will not be described herein. The aforementioned resin can be but not limited to an unsaturated polyester resin or a vinyl ester resin.

In the step 320, a curing step is performed, wherein the carbonate-containing unsaturated compound and the resin are performed the free radical copolymerization to form a crosslinked product, and a curing temperature of the curing reaction can be 25°C to 80°C. The curing temperature and the heating time can be appropriately adjusted according to the type of the used carbonate-containing unsaturated compound and the resin, and the present disclosure is not limited thereto.

### <A Method for Degrading a Crosslinked Product>

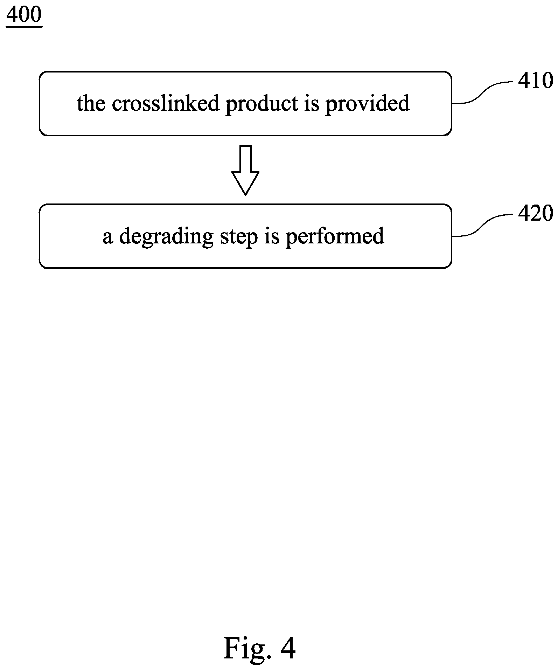

Reference is made to Fig. 4, which is a flow chart of a method for degrading a crosslinked product 400 according to yet another embodiment of the present disclosure. In Fig. 4, the method for degrading the crosslinked product 400 includes a step 410 and a step 420.

In the step 410, the aforementioned crosslinked product is provided. In the step 420, a degrading step is performed, wherein the aforementioned crosslinked product is degraded by using an amine compound as a degradation agent. Specifically, the aforementioned degrading step can be degraded at 80°C to 150°C without adding any catalyst, and the degradation agent after the degrading step can be purified by distillation, therefore the amine compound can be reused as the degradation agent again. Furthermore, the produced urea derivative can be recovered, and can be used in the coatings or the polyurethane materials to achieve the goal of recycling applications.

The present disclosure will be further exemplified by the following specific embodiments so as to facilitate utilizing and practicing the present disclosure completely by the people skilled in the art without over-interpreting and over-experimenting. However, the readers should understand that the present disclosure should not be limited to these practical details thereof, that is, these practical details are used to describe how to implement the materials and methods of the present disclosure and are not necessary.

### <Example and Comparative Example>

### <Preparation of Carbonate-Containing Unsaturated Compound>

Example 1: 10 g of dimethyl carbonate and 28.89 g of 2-hydroxyethyl methacrylate (HEMA) are mixed at the equivalence ratio of 1:1 to form the reactant. Next, adding trioctylmethylphosphonium methyl carbonate (P₈₈₈₁CH₃OCOO) ionic liquid of 0.5 weight percent of the total amount of the reactant, and heating to 80°C for 8 hours to obtain the carbonate-containing unsaturated compound of Example 1, and the yield thereof is about 60%. Data from FTIR spectra: 1748 cm⁻¹ (carbonate C=O), 1715 cm⁻¹ (acrylate C=O). The reaction equation of Example 1 is shown in Table 1.

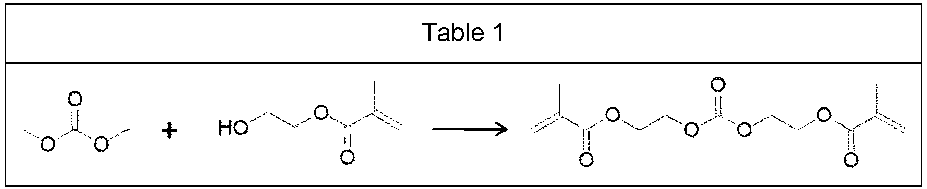

Example 2: 10 g of diphenyl carbonate and 13.27 g of glycidyl methacrylate (GMA) are mixed at the equivalence ratio of 1:1, and in the nitrogen environment at 110°C to form the homogeneous solution. Next, adding 0.027 g of pyridine (0.2 wt% of GMA) and reacting for 3 hours to obtain the carbonate-containing unsaturated compound of Example 2, and the yield thereof is about 90%. Data from FTIR spectra: 1749 cm⁻¹ (aliphatic carbonate C=O), 1715 cm⁻¹ (acrylate C=O). The reaction equation of Example 2 is shown in Table 2.

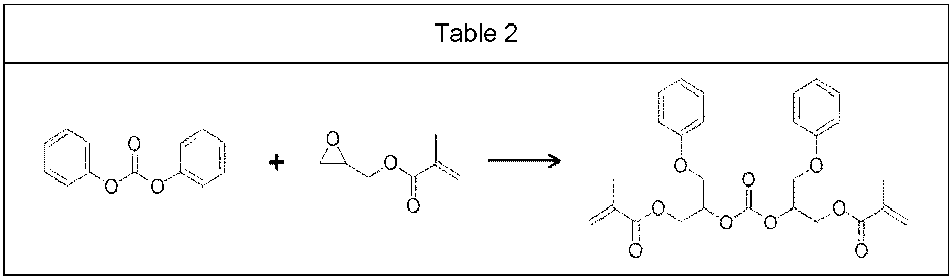

Example 3: 10 g of diphenyl carbonate and 34.54 g of bisphenol A diglycidyl ether (commodity code BE188 from CHANG CHUN PLASTICS CO., LTD.) are mixed at the equivalence ratio of 1:2, and in the nitrogen environment at 110°C to form the homogeneous solution. Next, adding 0.1727 g of pyridine (0.5 wt% of BE188) and reacting for 3 hours. Then, adding 8.04 g of methacrylic acid, wherein the equivalence ratio of the methacrylic acid to BE188 is 0.5:1, and reacting for 4 hours to obtain the carbonate-containing unsaturated compound of Example 3. Data from FTIR spectra: 1749 cm⁻¹ (carbonate C=O), 1719 cm⁻¹ (acrylate C=O). The reaction equation of Example 3 is shown in Table 3.

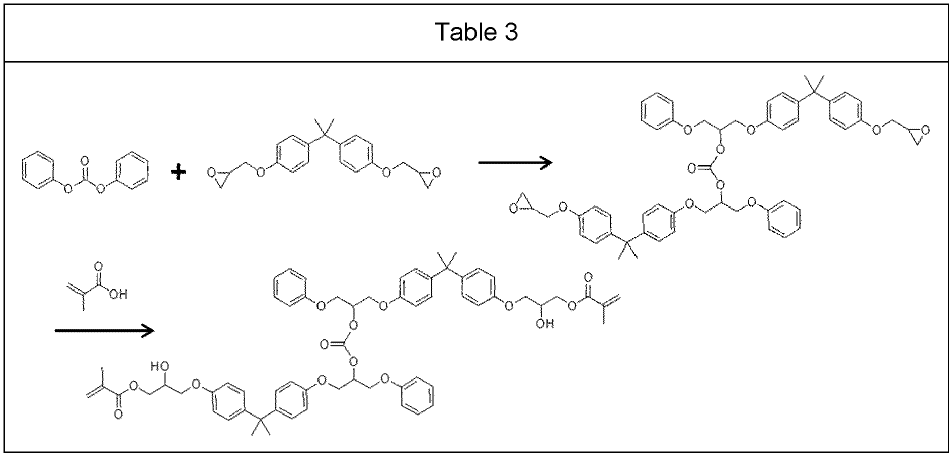

### <Preparation of Crosslinked Product>

The carbonate-containing unsaturated compound of Example 1 to Example 3 are adding to the unsaturated polyester resin (UP1) or bisphenol A vinyl ester resin (VE1), respectively. Next, adding styrene (SM) to dilute and adding 1 phr of peroxide MEKPO and 1 phr of cobalt octoate, then stirring evenly and pouring into the mold to cure at the room temperature for 12 hours. Then, curing at 80°C for 4 hours to obtain the crosslinked product of Example 4 to Example 9 and Comparative Example 1 to Comparative Example 4.

The formulation and the content used for Example 4 to Example 9 and Comparative Example 1 to Comparative Example 4 are shown in Table 4.

| Table 4 | | | | | | |
|---|---|---|---|---|---|---|
| | Example 1 | Example 2 | Example 3 | UP1 | VE1 | SM |
| Example 4 | 15 | 0 | 0 | 100 | 0 | 5 |
| Example 5 | 0 | 10 | 0 | 100 | 0 | 8 |
| Example 6 | 0 | 0 | 8 | 100 | 0 | 14 |
| Example 7 | 15 | 0 | 0 | 0 | 100 | 5 |
| Example 8 | 0 | 10 | 0 | 0 | 100 | 8 |
| Example 9 | 0 | 0 | 8 | 0 | 100 | 14 |
| Comparative Example 1 | 0 | 0 | 0 | 100 | 0 | 10 |
| Comparative Example 2 | 0 | 0 | 0 | 0 | 100 | 10 |
| Comparative Example 3 | 0 | 0 | 2 | 100 | 0 | 10 |
| Comparative Example 4 | 0 | 0 | 22 | 100 | 0 | 25 |

### <Thermal Property Evaluation>

Example 4 to Example 9 and Comparative Example 1 to Comparative Example 4 are performed the thermal property evaluation, which uses the differential scanning calorimeter (DSC) to measure the glass transition temperature (T_{g}) at a heating rate of 10°C/min, and the measurement results of the glass transition temperature (°C) are shown in Table 5.

| Table 5 | | | | | |
|---|---|---|---|---|---|
| | Example 4 | Example 5 | Example 6 | Example 7 | Example 8 |
| T_{g} | 115 | 114 | 117 | 123 | 120 |

| | Example 9 | Comparative Example 1 | Comparative Example 2 | Comparative Example 3 | Comparative Example 4 |
|---|---|---|---|---|---|
| T_{g} | 125 | 109 | 120 | 111 | 73 |

As shown in Table 5, the glass transition temperature of Comparative Example 4 is lower because the molecular weight of the carbonate-containing unsaturated compound of Example 3 is larger and the viscosity is higher, so that after adding Example 3, the mixing viscosity will increase significantly. Therefore, if the addition ratio of Example 3 is too high, it will lead to the need to add more diluent SM to achieve the required operating viscosity that is unfavorable for the physical properties of the crosslinked product. However, the suitable operating viscosities of Example 4 to Example 9 are achieved by the adjustment of the addition ratio of the diluent SM and the carbonate-containing unsaturated compound, and the good glass transition temperature is maintained.

### <Degradation of Crosslinked Product>

The crosslinked product of the present disclosure can be performed the degradation reaction by the amine compound. First, 0.2 g of the crosslinked product of Example 4 to Example 9 and Comparative Example 1 to Comparative Example 4 respectively and 4 g of hexylamine are placed in the container, and heated to 130°C in the oven. After heating for 24 hours, the remaining solid is taken out to measure the residual amount, and the residual weight (%) is listed in Table 6.

| Table 6 | | | | | |
|---|---|---|---|---|---|
| | Example 4 | Example 5 | Example 6 | Example 7 | Example 8 |
| residual weight | 0 | 0 | 0 | 0 | 0 |

| | Example 9 | Comparative Example 1 | Comparative Example 2 | Comparative Example 3 | Comparative Example 4 |
|---|---|---|---|---|---|
| residual weight | 0 | 78 | 95 | 54 | 0 |

As shown in Table 6, Example 4 to Example 9 can effectively provide a degradation site by introducing the carbonate group into the network structure, so that the disintegration efficiency of the network structure can be significantly increased, and the effect of complete degradation can be achieved at 130°C without the catalyst. Furthermore, Comparative Example 1 and Comparative Example 2 do not add the carbonate-containing unsaturated compound of the present disclosure, so that the degradation effect thereof is not good. However, in Comparative Example 3, due to the content of the carbonate-containing unsaturated compound is low, the purpose of complete degradation is not achieved, but it is still observed that the addition of the carbonate-containing unsaturated compound of the present disclosure has a significant improvement in degradability.

In conclusion, the carbonate-containing unsaturated compound of the present disclosure is obtained by using the carbonate compound and the epoxy compound or the alcohol compound that contains unsaturated double bond, which can be introduced into the commercially unsaturated polyester resin or vinyl ester resin for enhancing the degradability of the crosslinked product. Furthermore, the crosslinked product can be degraded by using the amine compound under the mild condition to response to the issue of the thermoset recycling.

## Claims

1. A carbonate-containing unsaturated compound, **characterized in** comprising a structure represented by formula (I) or formula (II): 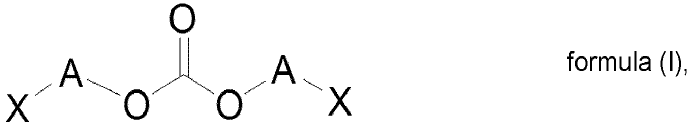 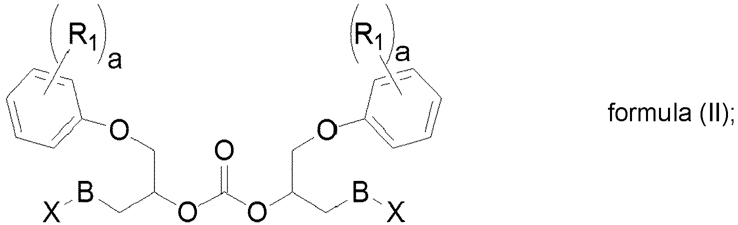
wherein X is a vinyl group, an allyl group, an acrylate or a methacrylate;
wherein R₁ is an alkyl group of 1 to 4 carbon atoms, a methoxy group, a nitro group or a halogen atom, and a is an integer from 0 to 5;
wherein A is an alkyl group of 1 to 12 carbon atoms, an alkoxy group of 1 to 12 carbon atoms or a structure represented by formula (i): 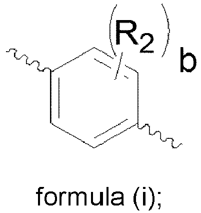
wherein R₂ is the alkyl group of 1 to 4 carbon atoms, the methoxy group, the nitro group or the halogen atom, and b is an integer from 0 to 4;
wherein B is the alkyl group of 1 to 12 carbon atoms, the alkoxy group of 1 to 12 carbon atoms, the structure represented by formula (i), or a structure represented by formula (ii) or formula (iii): 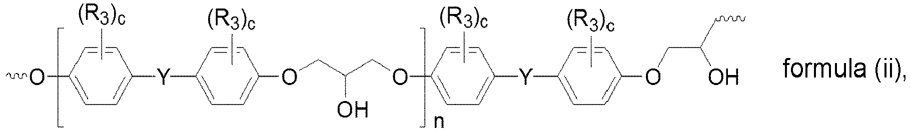 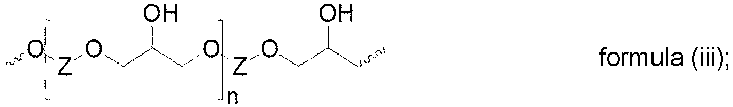
wherein R₃ is the alkyl group of 1 to 4 carbon atoms, the methoxy group, the nitro group or the halogen atom, and c is an integer from 0 to 4;
wherein Y is a single bond, the alkyl group of 1 to 12 carbon atoms, a cycloalkyl group of 3 to 12 carbon atoms, an oxygen atom, a sulfur atom, a sulfonyl group, a thionyl group, an acyl group, a fluorenyl group or a hexafluoropropane group;
wherein Z is the alkyl group of 1 to 12 carbon atoms, the alkoxy group of 1 to 12 carbon atoms, an otho-phenylene group, a meta-phenylene group or a para-phenylene group; and
wherein n is an integer from 0 to 10.

2. A manufacturing method for a carbonate-containing unsaturated compound (100), **characterized in** comprising:
providing a carbonate-containing compound (110), which is a dimethyl carbonate or a diphenyl carbonate;
providing an unsaturated double bond-containing compound (120), which is an unsaturated double bond-containing monofunctional alcohol compound or an unsaturated double bond-containing monofunctional epoxy compound; and
performing a catalyzing step (130), wherein the carbonate-containing compound is reacted with the unsaturated double bond-containing compound by using a catalyst to obtain a carbonate-containing unsaturated compound, which comprises a structure represented by formula (I) or formula (II): 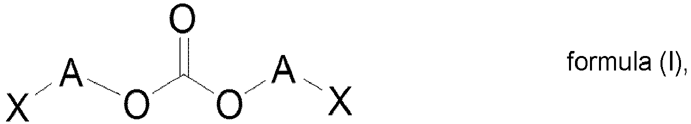 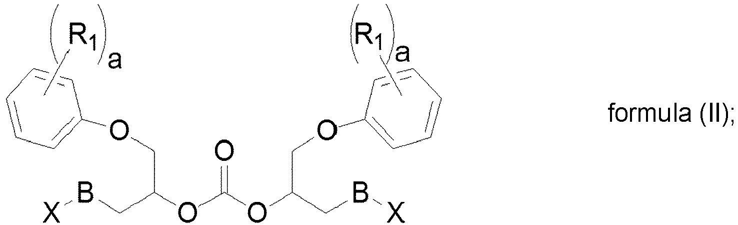
wherein X is a vinyl group, an allyl group, an acrylate or a methacrylate;
wherein R₁ is an alkyl group of 1 to 4 carbon atoms, a methoxy group, a nitro group or a halogen atom, and a is an integer from 0 to 5;
wherein A is an alkyl group of 1 to 12 carbon atoms, an alkoxy group of 1 to 12 carbon atoms or a structure represented by formula (i): 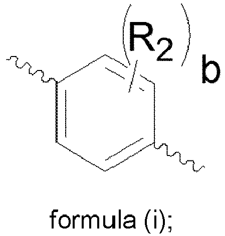
wherein R₂ is the alkyl group of 1 to 4 carbon atoms, the methoxy group, the nitro group or the halogen atom, and b is an integer from 0 to 4;
wherein B is the alkyl group of 1 to 12 carbon atoms, the alkoxy group of 1 to 12 carbon atoms, the structure represented by formula (i), or a structure represented by formula (ii) or formula (iii): 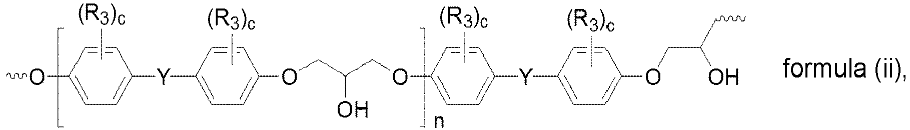 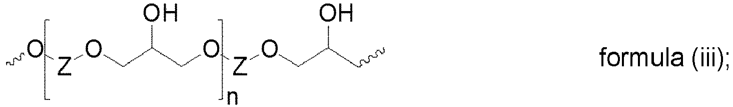
wherein R₃ is the alkyl group of 1 to 4 carbon atoms, the methoxy group, the nitro group or the halogen atom, and c is an integer from 0 to 4;
wherein Y is a single bond, the alkyl group of 1 to 12 carbon atoms, a cycloalkyl group of 3 to 12 carbon atoms, an oxygen atom, a sulfur atom, a sulfonyl group, a thionyl group, an acyl group, a fluorenyl group or a hexafluoropropane group;
wherein Z is the alkyl group of 1 to 12 carbon atoms, the alkoxy group of 1 to 12 carbon atoms, an otho-phenylene group, a meta-phenylene group or a para-phenylene group;
wherein n is an integer from 0 to 10.

3. The manufacturing method for the carbonate-containing unsaturated compound (100) of claim 2, wherein the catalyst is an ionic liquid or an organic base.

4. The manufacturing method for the carbonate-containing unsaturated compound (100) of claims 2 or 3, wherein an equivalence ratio of the carbonate-containing compound to the unsaturated double bond-containing compound is 0.8 to 1.2.

5. A manufacturing method for a carbonate-containing unsaturated compound (200), **characterized in** comprising:
performing a catalyzing step (210), wherein a diphenyl carbonate is reacted with a bifunctional epoxy compound by using a catalyst to obtain a reactant; and
performing an adding step (220), wherein an acrylic acid or a methacrylic acid is added in the reactant to obtain a carbonate-containing unsaturated compound, which comprises a structure represented by formula (II): 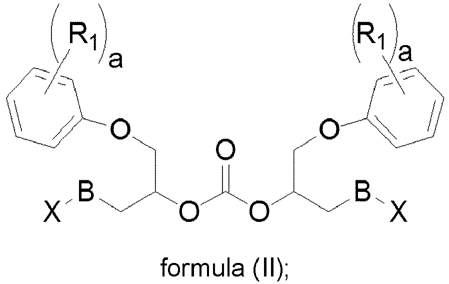
wherein X is a vinyl group, an allyl group, an acrylate or a methacrylate;
wherein R₁ is an alkyl group of 1 to 4 carbon atoms, a methoxy group, a nitro group or a halogen atom, and a is an integer from 0 to 5;
wherein B is an alkyl group of 1 to 12 carbon atoms, an alkoxy group of 1 to 12 carbon atoms, a structure represented by formula (i), formula (ii) or formula (iii): 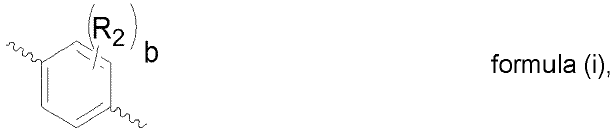 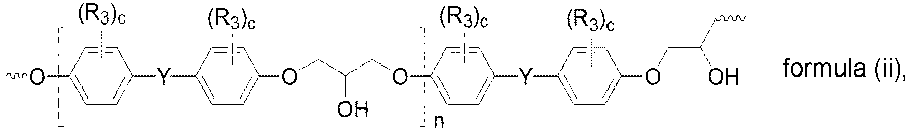 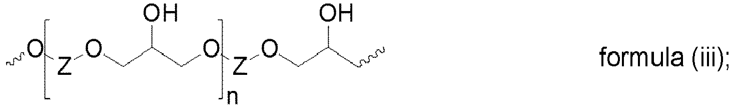
wherein R₂ is the alkyl group of 1 to 4 carbon atoms, the methoxy group, the nitro group or the halogen atom, and b is an integer from 0 to 4;
wherein R₃ is the alkyl group of 1 to 4 carbon atoms, the methoxy group, the nitro group or the halogen atom, and c is an integer from 0 to 4;
wherein Y is a single bond, the alkyl group of 1 to 12 carbon atoms, a cycloalkyl group of 3 to 12 carbon atoms, an oxygen atom, a sulfur atom, a sulfonyl group, a thionyl group, an acyl group, a fluorenyl group or a hexafluoropropane group;
wherein Z is the alkyl group of 1 to 12 carbon atoms, the alkoxy group of 1 to 12 carbon atoms, an otho-phenylene group, a meta-phenylene group or a para-phenylene group;
wherein n is an integer from 0 to 10.

6. The manufacturing method for the carbonate-containing unsaturated compound (200) of claim 5, wherein the catalyst is an organic base, and an equivalence ratio of the bifunctional epoxy compound to the diphenyl carbonate is 2.0 to 8.0.

7. A crosslinked product, which is obtained by performing a curing reaction with the carbonate-containing unsaturated compound of claim 1.

8. The crosslinked product of claim 7, wherein the curing reaction is completed by heating after the carbonate-containing unsaturated compound added to a resin.

9. The crosslinked product of claim 8, wherein the resin is an unsaturated polyester resin or a vinyl ester resin.

10. The crosslinked product of claims 8 or 9, wherein an added amount of the carbonate-containing unsaturated compound is ranged from 3% by weight to 20% by weight of an amount of the resin.

11. A method for degrading a crosslinked product (400), **characterized in** comprising:
providing the crosslinked product (410) of any one of claims 7 to 10; and
performing a degrading step (420), wherein the crosslinked product is degraded by using an amine compound as a degradation agent.
